Europäisches Patentamt

**European Patent Office**

**Office européen des brevets**

(19)

(11) Veröffentlichungsnummer: **0 032 252**
**B1**

(12) **EUROPÄISCHE PATENTSCHRIFT**

(45) Veröffentlichungstag der Patentschrift:
**22.12.82**

(51) Int. Cl.³: **C 07 H 15/04**

(21) Anmeldenummer: **80108258.7**

(22) Anmeldetag: **31.12.80**

(54) **Verfahren zur Reinigung von Alkylglycosiden durch destillative Abtrennung nicht umgesetzter Alkohole.**

(30) Priorität: **12.01.80 DE 3001064**

(43) Veröffentlichungstag der Anmeldung:
**22.07.81 Patentblatt 81/29**

(45) Bekanntmachung des Hinweises auf die Patenterteilung:
**22.12.82 Patentblatt 82/51**

(84) Benannte Vertragsstaaten:
**DE FR GB IT**

(56) Entgegenhaltungen:
**FR-A-1 364 548**
**FR-A-2 380 240**

(73) Patentinhaber: **BASF Aktiengesellschaft,
Carl-Bosch-Strasse 38, D-6700 Ludwigshafen (DE)**

(72) Erfinder: **Klahr, Ernst, Dr., Londoner Ring 11,
D-6700 Ludwigshafen (DE)**
Erfinder: **Trieselt, Wolfgang, Dr., Alwin-Mittasch-Platz 1,
D-6700 Ludwigshafen (DE)**
Erfinder: **Trapp, Horst, Dr.,
Johann-Sebastian-Bach-Strasse 10A, D-6831 Plankstadt
(DE)**
Erfinder: **Widder, Rudi, Dr., In der Taesch 7,
D-6906 Leimen (DE)**

EP 0 032 252 B1

Verfahren zur Reinigung von Alkylglycosiden durch destillative Abtrennung nicht umgesetzter Alkohole

Die Erfindung betrifft ein Verfahren zur Reinigung höherer Alkylglycoside durch destillative Abtrennung der bei der Herstellung nicht umgesetzten höheren Fettalkohole.

Höhere Alkylglycoside, die als biologisch abbaubare oberflächenaktive Stoffe eine vielseitige Verwendung finden, werden heute fast ausschliesslich durch Umsetzung von höheren Alkoholen mit niederen Alkyl- oder Hydroxyalkylglycosiden, welche letztere inwiederum durch Umsetzung von Monosacchariden oder von zu Monosacchariden hydrolysierbaren Verbindungen mit niederen Alkoholen entstehen, in Gegenwart saurer Katalysatoren hergestellt.

Bei dieser Methode, zu der zahlreiche Varianten bekannt sind – Literatur s.u. – entsteht in jedem Fall nach der Umsetzung eine Mischung aus dem höheren Alkylglycosid und dem entsprechenden nichtumgesetzten Alkohol, der entfernt werden muss. Da die Siedepunkte der höheren Alkohole, vornehmlich der Fraktionen mit mehr als 12 Kohlenstoffatomen sehr hoch liegen, musste man entweder reine Octyl- bis Dodecylalkohole zur Umsetzung bringen, um diese mittels einer Destillation unter stark vermindertem Druck bei Temperaturen unter 140°C – bei höheren Temperaturen zersetzen sich bereits die Zuckerreste unter Dunkelfärbung – problemlos abtrennen zu können, oder aber man musste ein extrem hohes Vakuum anlegen, wenn höhere Alkoholfraktionen, z.B. technische Gemische mit $C_{14}$- oder höheren Alkylresten, noch entfernt werden mussten.

Gerade aber, wenn man in Wasser klar lösliche Alkylglycoside erhalten will, müssen diese Alkylglycoside praktisch alkoholfrei sein, da sonst trübe Lösungen entstehen.

Die Herstellung von Alkylglycosiden auf Basis von technischen Alkoholgemischen mit Anteilen an höheren ($C_{14}$–$C_{16}$)-Alkoholen ist wegen der gegenüber der Herstellung mit reinen Alkoholen, wie Decyl- oder Dodecylalkohol, wesentlich grösseren Wirtschaftlichkeit vorzuziehen.

Da das für die Entfernung solcher höhere Alkoholanteile enthaltender Gemische erforderliche Hochvakuum den Prozess ebenfalls unwirtschaftlicher macht, musste man häufig einen Kompromiss derart suchen, dass die letzten Alkoholanteile im Gemisch verblieben oder bei Temperaturen über 140°C abdestilliert wurden, wodurch die Produkte sehr dunkel gefärbt waren.

Eine weitere Schwierigkeit, die der destillativen Abtrennung der höheren Alkohole entgegenwirkt, besteht darin, dass höhere Alkylglycoside bei Temperaturen von bis zu 140°C hochviskose Stoffe darstellen, was naturgemäss die Entfernung der letzten Alkoholanteile mit destillativen Mitteln fast unmöglich machte.

Das Ziel der Erfindung bestand in der Entwicklung einer Methode, die es ermöglicht, $C_8$- bis $C_{16}$-Alkylglycoside durch destillative Abtrennung auch der letzten nichtumgesetzten Alkoholanteile zu reinigen.

Dieses Ziel wurde mit einem Verfahren erreicht, wie es gemäss dem Patentanspruch definiert ist.

Die einzusetzenden Glycole wirken in zweierlei Hinsicht. Einmal wirken sie als Schleppmittel, d.h. sie ermöglichen die destillative Entfernung der Restalkohole bei Temperaturen von < 140°C und bei Drücken von ca. 8 Millibar, d.h. technisch unschwer realisierbaren Drücken, und zum anderen wirken sie als Lösungs(Verdünnungs-)mittel für die Alkylglycoside, die damit auch bei der Destillationstemperatur in niedrigviskoser Form vorliegen.

Die zu reinigenden Alkylglycoside leiten sich von reduzierend wirkenden Monosacchariden, wie Pentosen oder Hexosen oder von der zu derartigen Monosacchariden hydrolysierbaren Verbindungen ab. Beispiele für geeignete Monosaccharide sind Glucose, Mannose, Galactose, Talose, Allose, Altrose, Idose, Arabinose, Xylose, Ribose und Lyxose. Zu den zu Monosacchariden hydrolysierbaren reduzierend wirkenden Zuckern gehören z.B. Oligosaccharide und Polysaccharide, wie Maltose, Lactose, Saccharose, Raffinose, Dextrine, Stärken, Maissirup und Holzzucker.

Bevorzugt wird Glucose oder eine direkt zu Glucose hydrolysierbare Verbindung.

Alkohole, die den Alkylglycosiden zugrundeliegen, sind erfindungsgemäss Alkohole mit 8 bis 16 C-Atomen. Hierzu gehören z.B. Octanol, Decanol, Dodecanol, Tetradecanol und Hexadecanol sowie deren Mischungen. Bevorzugt sind technische Gemische, die aus der Oxo- und Ziegler-Synthese stammen, und zwar $C_9$–$C_{11}$-, $C_{13}$–$C_{15}$-oxo- und $C_{10}$–$C_{12}$-, $C_{12}$–$C_{14}$-, und $C_{14}$–$C_{16}$-Ziegler-Alkohole. Besonders bevorzugt, weil am wohlfeilsten, sind technische $C_{10}$–$C_{16}$-Alkoholgemische aus der Ziegler-Synthese.

Die Herstellung der Glycoside gehört zum Stand der Technik und geschieht z.B. gemäss GB-PS 1 072 655 in der Weise, dass man Glucose oder ein niederes Alkylgulcosid in Gegenwart eines sauren Ionenaustauschers mit einem der höheren Alkohole umsetzt.

Gemäss der DE-OS 19 05 523 erhält man Glycoside dadurch, dass man z.B. Glucose in Gegenwart eines niederen Alkohols oder Ätheralkohols in beispielsweise schwefelsaurem Medium mit den höheren Alkoholen umsetzt. Auch diese Reaktion läuft letzten Endes über eine Umacetalisierung eines niederen Alkylglycosides mit einem höheren Alkohol ab.

Aus der DE-AS 20 36 472 ist bekannt, dass statt der niederen Alkohole auch niedere Glycole eingesetzt werden können; intermediär und als Mischungspartner im Endprodukt treten somit Hydroxyalkylglycoside auf.

Schliesslich kann man auch den Zucker zunächst ausschliesslich mit einem niederen Glycol mit 3 bis 5 C-Atomen in saurem Medium umsetzen, das erhaltene Hydroxyalkylglycosid zwischenisolieren und dann, wieder in saurem Medium, mit dem gewünschten höheren Alkohol umsetzen.

Bei all diesen Methoden entstehen Glycoside oder Glycosidgemische mit grösseren Anteilen an unumgesetztem höheren Alkohol.

Nach der Neutralisation des sauren Katalysatoranteils werden diese höheren Alkohole destillativ entfernt. Hierbei setzt man erfindungsgemäss eines der anspruchsgemäss definierten Glycole entweder sofort oder zumindest bei der Entfernung der letzten Alkoholreste zu, wodurch Temperaturen über 140°C vermieden werden können. Vorzugsweise geht man so vor, dass man das aus einer der obengenannten Umsetzung stammende Glycosid-Alkoholgemisch zunächst einer Destillation unter vermindertem Druck ohne Zusatz unterwirft, wobei die höheren Alkohol-Anteile ($C_{14}$–$C_{16}$-Alkohole) zurückbleiben. Anschliessend setzt man das Glycol zu und destilliert bei ca. 8 mbar und 135 bis 140°C, gegebenenfalls mehrmals, bis eine Probe des Rückstandes in Wasser klar löslich ist. Die Menge des zuzusetzenden Glycols richtet sich nach der Menge des abzutrennenden Restalkohols – sie liegt im allgemeinen – bezogen auf das Gewicht des Alkylglycosides – bei 5 bis 100%, vorzugsweise 10 bis 50%.

Die zuzusetzenden Glycole sollen Siedepunkte besitzen, welche den des abzudestillierenden Restalkohols um nicht mehr als 10 Grad über- und nicht mehr als 30° unterschreiten. Die besten Ergebnisse erhält man bei Differenzen von <5 Grad.

Derartige Glycole sind beispielsweise Butandiol-1,4, Diäthylenglykol, Dipropylenglykol, Dibutylenglykol und Neopentylglykol sowie Gemische dieser Glycole.

Im folgenden Beispiel wird die Erfindung erläutert.

Beispiel

Aus 70,13 kg eines $C_{10}$–$C_{12}$-Alkylglucosides (hergestellt aus Glucose und einem Alkoholschnitt, dessen Hauptanteile ein $C_{10}$–$C_{12}$-Schnitt war und der Anteile von Alkoholen mit bis zu 16 C-Atomen enthielt), das noch 54 Gew.-% an unumgesetztem Alkoholgemisch enthielt, wurde zunächst bei 140°C und 8 mbar die Hauptmenge des überschüssigen Alkohols abdestilliert. Anschliessend wurden dem Destillationsrückstand 10 kg Dipropylenglycol in mehreren Portionen zugesetzt und jeweils auf 140°C bei 8 mbar erhitzt, wobei die letzten Alkoholreste zusammen mit dem Dipropylenglykol abdestillierten.

Ein Parallelversuch ohne Zusatz bei einem Druck von 1,5 mbar und 150°C ergab ein dunkelbraunes Produkt, das in Wasser nur trübe löslich war.

**Patentanspruch**

Verfahren zur Reinigung von $C_8$- bis $C_{16}$-Alkylglycosiden, die durch Reaktion von niederen Alkylglycosiden oder Hydroxyalkylglycosiden mit $C_8$- bis $C_{16}$-Alkoholen in Gegenwart von sauren Katalysatoren erhalten werden, durch destillative Abtrennung nicht umgesetzter $C_8$- bis $C_{16}$-Alkohole, dadurch gekennzeichnet, dass man die destillative Abtrennung zumindest der letzten Anteile an nichtumgesetzten Alkoholen in Gegenwart von Glycolen durchführt, deren Siedepunkte die der abzutrennenden Alkohole um höchstens 10 Grad über- und um höchstens 30 Grad unterschreiten.

**Claim**

A process for purifying $C_8$–$C_{16}$-alkylglycosides which have been obtained by reacting lower alkylglycosides or hydroxyalkylglycosides with $C_8$–$C_{16}$-alcohols in the presence of an acid catalyst, by distillative removal of unconverted $C_8$–$C_{16}$-alcohols, wherein the distillative removal, at least of the last amounts of unconverted alcohols, is carried out in the presence of glycols whose boiling points are not more than 10° above, and not more than 30° below, those of the alcohols to be removed.

**Revendication**

Procédé de purification d'alkylglycosides en $C_8$ à $C_{16}$ obtenus par réaction d'alkylglycosides inférieurs ou d'hydroxyalkylglycosides avec des alcools en $C_8$ à $C_{16}$, en présence de catalyseurs acides, par séparation distillatoire d'alcools en $C_8$ à $C_{16}$ n'ayant pas réagi, caractérisé par le fait qu'on effectue la séparation distillatoire, au moins des dernières portions d'alcools n'ayant pas réagi, en présence de glycols dont le point d'ébullition n'est pas supérieur de plus de 10 degrés et n'est pas inférieur de plus de 30 degrés à celui des alcools à séparer.